# DEMANDE DE BREVET EUROPEEN

(11) **EP 1 087 251 A1**
(43) Date de publication de la demande: **28.03.2001**
(21) Numéro de dépôt: 00402584.7
(22) Date de dépôt: 19.09.2000
(51) Int. Cl.: G02C 7/04, A61B 3/10, B24B 13/06, G05B 19/18, A61B 3/103, A61B 3/107

(54) **Procédé et dispositif pour déterminer la forme d'une lentille de contact**

(30) Priorité: 22.09.1999 FR 9911843
(71) Demandeur: ESSILOR INTERNATIONAL (Compagnie Générale d'Optique), F-94220 Charenton-le-Pont (FR)
(72) Inventeur: Chateau, Nicolas, 75013 Paris (FR); Marin, Gildas, 92160 Antony (FR); Fermigier, Bruno, 75014 Paris (FR)
(74) Mandataire: CABINET BONNET-THIRION

(57) **Abrégé**

Le procédé comporte:
- une étape de mesure de la topographie de la cornée de l'oeil à corriger,
- une étape de détermination de la forme de la face postérieure de la lentille à partir de la topographie mesurée de la cornée pour obtenir un comportement mécanique prédéterminé de la lentille lorsqu'elle est posée sur l'oeil,
- une étape de mesure des aberrations optiques de l'oeil à corriger,
- une étape de détermination de la forme de la face antérieure de ladite lentille pour corriger ces aberrations, à partir de la mesure des aberrations optiques de l'oeil à corriger combinée à des données relatives à la forme déterminée pour la face postérieure de la lentille.

Le dispositif comporte des unités de mesure de la topographie (1) et des aberrations (2) de l'oeil et une unité électronique de calcul (3).

## Description

La présente invention concerne de manière générale la conception des lentilles de contact ophtalmiques et plus précisément un procédé pour déterminer la forme d'une lentille de contact ophtalmique capable de corriger les aberrations optiques de l'oeil au-delà de la défocalisation ou de l'astigmatisme. Elle concerne également un dispositif pour mettre en oeuvre ce procédé.

Les lentilles de contact ophtalmiques actuelles, malgré tous les progrès apportés à leur conception, se contentent de corriger les défauts optiques les plus grossiers de l'oeil que constituent la défocalisation et l'astigmatisme. Or, on sait depuis longtemps que l'oeil peut présenter des défauts supplémentaires résultant d'aberrations optiques d'ordre supérieur, plus fines, affectant les différents composants de l'oeil : cornée, cristallin ou milieux intraoculaires. Ces aberrations optiques d'ordre supérieur de l'oeil brouillent l'image formée sur la rétine, ce qui perturbe la vision alors même que les éventuels défauts de défocalisation ou d'astigmatisme sont corrigés. Dans certains cas pathologiques de cornée irrégulière, comme par exemple le kératocone, ces aberrations rendent pratiquement impossible la vision des formes.

Récemment, des techniques de mesures ophtalmiques ont été développées pour mesurer avec précision, en plus des défauts classiques de défocalisation ou d'astigmatisme, des aberrations optiques d'ordre supérieur de l'oeil. C'est ainsi que le brevet US 5 777 719 propose un procédé et un dispositif pour mesurer avec précision les aberrations optiques de l'oeil par l'analyse du front d'onde d'un laser réfléchi par la rétine de l'oeil d'après la méthode Hartmann-Shack. A partir de cette mesure, il est théoriquement possible de déterminer la forme d'une lentille de contact ophtalmique de façon à compenser les aberrations mesurées. Cette technique constitue une personnalisation de la lentille aux aberrations optiques particulières de l'oeil du patient.

On a donc pu penser à un procédé pour déterminer la forme d'une lentille de contact ophtalmique de correction des aberrations optiques d'un l'oeil, comportant une étape de mesure des aberrations optiques de l'oeil à corriger, et une étape de détermination de la forme de ladite lentille à partir de la mesure des aberrations optiques de l'oeil à corriger pour corriger ces aberrations.

On rappelle que la face antérieure d'une lentille de contact est la face convexe de cette lentille qui est opposée à l'oeil, tandis que la face postérieure d'une lentille est la face concave de cette lentille qui est au contact de l'oeil.

Toutefois, à ce stade, la demanderesse a constaté qu'une lentille conçue à partir de ces seules données ne réalise pas la compensation espérée lorsqu'elle est en pratique installée sur l'oeil du patient.

En effet, les lentilles présentent en général une face arrière de forme simple dont seul le rayon de courbure central est adapté à la surface de la cornée. Or la cornée a, le plus souvent, une forme complexe et variable d'un individu à l'autre. L'écart de forme entre la cornée de la face arrière de la lentille produit plusieurs effets. Cet écart provoque des déformations de la lentille et créer un film de larmes d'épaisseur irrégulière, entre la lentille et la cornée. Ces deux effets induisent des aberrations parasites supplémentaires qui dégradent les performances visuelles.

On connaît par ailleurs une autre forme de personnalisation des lentilles de contact ophtalmiques. On sait en effet que la surface de la cornée n'est pas parfaitement régulière mais est au contraire généralement asymétrique et asphérique. Selon son degré et sa nature, une telle irrégularité de la surface de la cornée peut se révéler source de conflit entre cette surface et la surface postérieure de la lentille de contact qui, elle, est généralement parfaitement régulière, de type sphérique, asphérique de révolution ou torique. Il en résulte une gêne pour le porteur qui conduit parfois à des irritations de la cornée et à un rejet de la lentille, forçant le patient à utiliser des lunettes de préférence aux lentilles de contact. Pour remédier à ce problème, il a été proposé, comme par exemple dans le brevet US 5 570 142, de réaliser des lentilles de contact dont la face postérieure est "adaptée" à la surface irrégulière de la cornée. Cette "adaptation" consiste en une correspondance de forme entre la surface postérieure de la lentille et la surface de la cornée, qui peut être complète, sur toute la surface postérieure de la lentille, ou seulement partielle, localisée en périphérie de la lentille, de manière à réaliser une assise stable et régulière de la lentille sur la cornée.

Mais quelle que soit la manière dont la surface postérieure de la lentille est adaptée à la surface de la cornée, cette adaptation personnalisée a pour seule fonction d'améliorer le confort du porteur ou d'empêcher la rotation de la lentille sur l'oeil, sans prendre en compte les aberrations oculaires

Dans ce contexte, l'invention a pour but de concevoir un procédé pour déterminer la forme d'une lentille de contact ophtalmique personnalisée, capable, lorsqu'elle est effectivement installée sur l'oeil du patient, de corriger les aberrations d'ordre supérieur mesurées sur cet oeil en limitant la génération d'aberrations parasites.

Selon l'invention, on propose un procédé pour déterminer la forme d'une lentille de contact ophtalmique de correction des aberrations optiques d'un oeil, comportant :
- une étape de mesure des aberrations optiques de l'oeil à corriger,
- une étape de détermination de la forme de la face antérieure de ladite lentille à partir de la mesure des aberrations optiques de l'oeil à corriger pour corriger ces aberrations,
caractérisé en ce qu'il comporte :
- une étape de mesure de la topographie de la cornée de l'oeil à corriger, et
- une étape de détermination de la forme de la face postérieure de la lentille à partir de la topographie mesurée de la cornée pour limiter la génération d'aberrations parasites lorsque la lentille est posée sur l'oeil,
et en ce que, pour la détermination de la forme de la face antérieure de la lentille, des données relatives à la forme déterminée pour la face postérieure de la lentille sont combinées à la mesure des aberrations optiques de l'oeil.

Connaissant le front d'onde à corriger, la forme de la face postérieure, la longueur d'onde, l'indice du matériau et l'épaisseur au centre, on peut calculer la forme requise pour la face antérieure en résolvant le problème inverse de tracé de rayons en trois dimensions.

Ce procédé prend en compte une contrainte d'importance pratique fondamentale qui n'avait pas encore pu être identifiée à ce jour dans le cadre de la correction des aberrations optiques d'ordre supérieur de l'oeil. En effet, pour corriger les aberrations d'ordre supérieur de l'oeil au moyen d'une lentille de contact, il ne suffit pas de mesurer ces aberrations et de concevoir la forme de la face antérieure de la lentille pour qu'elle compense ces aberrations. Il faut en outre éviter, ou tout au moins maîtriser pour les prendre en compte, les déformations de la lentille sur l'oeil ainsi que les irrégularités de l'épaisseur de larme entre la cornée et la lentille. Le fait que, selon le procédé de l'invention, la face postérieure de la lentille soit conçue de façon personnalisée, permet, à partir de la topographie mesurée de la cornée, de maîtriser ces deux paramètres essentiels que constituent la déformation de la lentille et l'épaisseur de larme. On comprend en effet que de ces deux paramètres dépend la correction optique effective réalisée par la lentille. Leur maîtrise est donc essentielle dans le cadre de la correction des aberrations d'ordre supérieur de l'oeil dans la mesure où cette correction est par nature beaucoup plus fine qu'une simple correction de la défocalisation ou de l'astigmatisme et nécessite par conséquent une précision élevée de la forme de la lentille, non seulement in vitro, c'est-à-dire lors de sa fabrication, mais également in vivo, c'est-à-dire lorsqu'elle est en place sur l'oeil du patient.

Le comportement mécanique de la lentille in vivo est ainsi maîtrisé par une conception particulière de la face postérieure de la lentille. La forme particulière de cette face doit être prise en compte dans la détermination de la face antérieure de la lentille qui détermine en dernier lieu la correction optique globalement procurée par la lentille. En effet, de la forme de la face postérieure de la lentille dépendent, d'une part les caractéristiques optiques de cette lentille elle-même, mais également l'épaisseur de larme, qui est éventuellement irrégulière (mais maîtrisée), et qui influe de manière déterminante sur la correction optique globalement obtenue.

La forme de la face postérieure de la lentille pourra par exemple être conçue de façon à ce qu'il existe un film de larmes équi-épais entre la lentille de la cornée. Dans un autre mode de réalisation, cette face pourra être conçue de façon à ce que l'épaisseur du film de larmes augmente lorsque la distance par rapport au centre de la lentille augmente.

De préférence, la mesure de la topographie de la cornée de l'oeil à corriger et la mesure des aberrations dudit oeil à corriger sont réalisées dans un référentiel spatial commun. Plus précisément, le référentiel spatial commun possède un premier axe confondu avec la ligne principale de visée de l'oeil à corriger, un second axe horizontal et orthogonal au premier axe et un troisième axe orthogonal aux premier et second axes. La ligne principale de visée correspond à la ligne passant par le point de fixation dans l'instrument et par le centre de la pupille d'entrée de l'oeil.

Selon un autre aspect avantageux de l'invention, on pourra prévoir que, pour améliorer encore la correction optique, notamment chez certains patitents où une précision accrue est exigée, le procédé comporte :
- une étape de fabrication d'une lentille d'essai ayant des faces antérieure et postérieure conformes aux déterminations obtenues à l'issue des étapes précitées,
- une étape de mesure in vivo des aberrations optiques de l'ensemble optique constitué par l'oeil à corriger équipé de la lentille d'essai fabriquée, et
- une étape de correction de la forme de la face antérieure de la lentille à partir de données relatives à la forme initialement déterminée de la lentille d'essai et de la nouvelle mesure des aberrations optiques de l'oeil avec la lentille d'essai.

L'invention a également pour objet un dispositif pour la mise en oeuvre d'un procédé du type précité, ce dispositif comportant :
- une unité de mesure de la topographie de la cornée de l'oeil à corriger, délivrant des données numériques représentatives de cette topographie,
- une unité de mesure des aberrations de l'oeil à corriger, délivrant des données numériques représentatives de ces aberrations,
- une unité électronique de calcul apte à déterminer, à partir des données qui lui sont délivrées d'une part par l'unité de mesure de la topographie de la cornée de l'oeil à corriger et d'autre part par l'unité de mesure des aberrations dudit oeil à corriger, les formes des faces antérieure et postérieure de la lentille, et à délivrer des données numériques représentatives de ces formes.

Selon un autre aspect avantageux de l'invention, le procédé peut être simplifié dans certains cas particuliers de pathologie, en prévoyant que l'étape de mesure des aberrations de l'oeil à corriger se confond avec l'étape de mesure de la topographie de la cornée dudit oeil à corriger, ces aberrations étant déduites par le calcul de la topographie mesurée de ladite cornée.

Ce type de procédé peut être avantageux dans les cas où les aberrations de la cornée sont prépondérantes, ce qui a déjà été observé.

Dans ce cas, le dispositif pour mettre en oeuvre un tel procédé comporte :
- une unité de mesure de la topographie de la cornée de l'oeil à corriger, délivrant des données numériques représentatives de cette topographie,
- une unité électronique de calcul apte à estimer les aberrations produites par la cornée à partir des données délivrées par l'unité de mesure de la topographie de la cornée, à déterminer les formes des faces antérieure et postérieure de la lentille à partir des données qui lui sont délivrées par l'unité de mesure de la topographie de la cornée et des aberrations estimées et à délivrer des données numériques représentatives de ces formes.

Avantageusement, l'un ou l'autre de ces dispositifs peut également comporter une unité de fabrication d'une lentille à partir des données numériques qui lui sont délivrées par l'unité de calcul. On obtient ainsi un dispositif complet, pouvant être entièrement automatisé, de fabrication d'une lentille de contact personnalisée.

D'autres caractéristiques et avantages de l'invention apparaîtront à la lecture de la description qui suit de modes de réalisation particuliers donnés à titre d'exemples non limitatifs.

Il sera fait référence aux dessins en annexe, parmi lesquels :

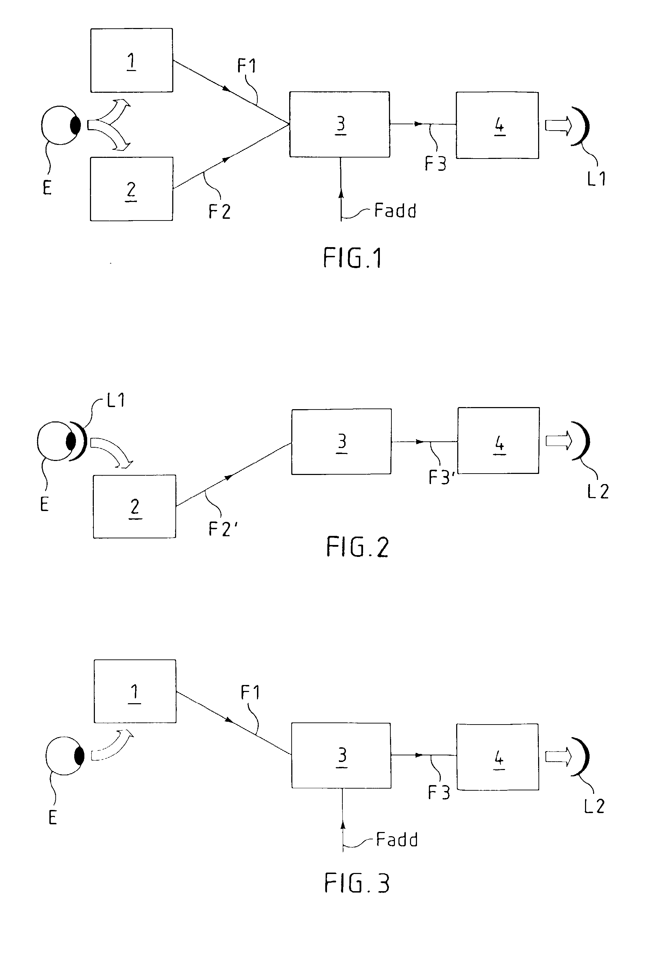

- la figure 1 est un diagramme illustrant un dispositif pour la fabrication d'une lentille de contact à partir des mesures de la topographie de la cornée et des aberrations optiques de l'oeil à corriger, mettant en oeuvre un premier mode de réalisation du procédé et du dispositif de détermination de la forme d'une lentille selon l'invention ;
- la figure 2 est un diagramme illustrant la mise en oeuvre du dispositif de la figure 1 pour affiner la forme de la lentille à partir de la mesure des aberrations optiques de l'oeil équipé d'une lentille d'essai déterminée conformément au diagramme de la figure 1 ;
- la figure 3 est un diagramme illustrant un dispositif pour la fabrication d'une lentille de contact mettant en oeuvre un second mode de réalisation, simplifié, du procédé et du dispositif de détermination de la forme d'une lentille selon l'invention, dans lequel les mesures de la topographie de la cornée et des aberrations optiques de l'oeil à corriger sont réalisées en une seule étape et par la seule unité de mesure de la topographie de la cornée.

En référence à la figure 1, un dispositif pour la détermination de la forme d'une lentille de contact ophtalmique de correction des aberrations d'ordre supérieur de l'oeil comporte :
- une unité de mesure 1 de la topographie de la cornée de l'oeil E à corriger, délivrant des données numériques représentatives de cette topographie,
- une unité de mesure 2 des aberrations de l'oeil E à corriger, délivrant des données numériques représentatives de ces aberrations,
- une unité électronique de calcul 3 apte à déterminer, à partir des données qui lui sont délivrées d'une part par l'unité de mesure 1 de la topographie de la cornée de l'oeil à corriger et d'autre part par l'unité de mesure 2 des aberrations dudit oeil à corriger, les formes des faces antérieure et postérieure de la lentille, et à délivrer des données numériques représentatives de ces formes.

Ce dispositif est complété par une unité de fabrication 4 d'une lentille de contact L1 à partir des données délivrées par l'unité de calcul 3. On obtient ainsi un dispositif complet, entièrement automatisé, pour la fabrication de lentilles de contact personnalisées.

L'unité de mesure 1 de la topographie de la cornée peut revêtir différents modes d'exécution qui sont tous connus et disponibles dans le commerce. On pourra par exemple utiliser les dispositifs suivants :
- topograhes à disques de Placido: Tomey TMS-1 (Computed Anatomy), EyeSys Corneal Analysis System (EyeSys Laboratories),
- topograhes à triangulation (rasterstereography): PAR Technology Corneal Topography System (PAR Technology), Orbscan (Orbtek).

Les différents dispositifs existant pour la mesure de la topographie cornéenne sont présentés dans l'article suivant : T. Dave, "Current developments in measurement of corneal topography", Contact lens and Anterior Eye 21, pp. S13-S30 (1998).

Quel que soit le dispositif choisi pour sa constitution, l'unité de mesure 1 de la topographie de la cornée doit fournir des données sur la plus grande partie possible de la surface de l'oeil qui sera recouverte par la lentille. Il est souhaitable d'obtenir la répartition d'élévation de la cornée et d'une partie de la sclère. Cela est possible notamment avec le Maastricht Shape Topôgrapher, qui est un dispositif utilisant la projection de franges de Moiré, commercialisé aux Etats-Unis d'Amérique et décrit dans les documents suivants :
- Jongsma F.H.M., Laan F.C., Stultiens B.A.T., "A Moiré based corneal topographer suitable for discrete Fourier analysis", SPIE Vol. 2126, Ophthalmic Technoloy 4, 1994 : 185-92. M.C ;
- Corbett, D.P. O'Brart, B.A. Stultiens, F.H. Jongsma, J.Marshall, "Corneal topography using a new moiré image-based system", Eur. J. Implant Ref. Surg. 7, 353-70 (1995).

Tous ces topographes sont capables de fournir un fichier électronique contenant, sous forme numérique, les coordonnées de points de la cornée.

L'unité de mesure 2 des aberrations optiques de l'oeil E peut elle aussi être réalisée de différentes manières. On pourra en particulier utiliser :
- un dispositif à méthode subjective du type décrit dans les documents suivants :
   - M.S. Smirnov, "Measurement of the wave aberration of the human eye". Biophysics 6, 776-794 (1961) ;
   - He JC, Marcos S, Webb RH, Burns SA. "Measurement of the wave-front aberration of the eye by a fast psychophysical procedure", J. Opt. Soc. Am. A15 : (9)2449-2456 (1998) ;
- un dispositif utilisant la méthode du test de Foucault, du type décrit dans le document suivant : F. Berny and S. Slansky, "Wavefront determination resulting from foucault test as applied to the human eye and visual instruments", in Optical Instruments and Techniques, J.H. Dickenson, ed. (Oriel, Newcastle, UK, 1969), pp. 375-386;
- un aberroscope, tel que décrit dans le document suivant : H.C. Howland and B. Howland, "A subjective method for the measurement of monochromatic aberrations of the eye", J. Opt.Soc.Am. 67, 1508-1518, (1977) ;
- un dispositif à capteur de front d'onde d'Hartmann-Shack du type décrit dans les documents suivants :
   - "A new method to precisely measure the wave aberrations of the human eye with a Hartmann-Shack wavefront sensor", Phd. Dissertation, J. Liang, University of Heidelberg (1991) ;
   - J. Liang, B. Grimm, S. Goelz, & J.F. Bille, "Objective measurement of Was of the human eye with the us of a Hartmann-Shack wave-front sensor", J. Opt. Soc. Am. A. 11, 1949-1957 (1994) ;
   - J. Liang, and D.R. Williams, "Aberrations and retinal image quality of the normal human eye", J. Opt. Soc. Am. A 14, 2873-2883 (1997).

Il pourra être particulièrement avantageux d'utiliser pour l'unité de mesure 2 des aberrations optiques de l'oeil un dispositif à capteur de front d'onde d'Hartmann-Shack, comme mentionné ci-dessus en dernier lieu, dans la mesure où la méthode mise en oeuvre dans ce dispositif est totalement objective et relativement simple et rapide à mettre en oeuvre.

Tous ces dispositifs sont bien entendu capables de fournir un fichier contenant des données numériques représentatives des aberrations oculaires mesurées de l'oeil.

L'unité électronique de calcul 3 peut par exemple être réalisée sous la forme d'un micro-ordinateur fonctionnant selon un logiciel dont les fonctionnalités seront décrites ultérieurement.

L'unité de fabrication 4 peut par exemple être constituée par une machine à commande numérique d'usinage asymétrique direct à précision sub-micronique, utilisée actuellement pour la fabrication des lentilles de contact. On pourra également utiliser d'autres procédés, comme par exemple la création de gradient d'indice.

Quel que soit le procédé de fabrication et la machine utilisés, l'unité de fabrication 4 est en tout état de cause de type numérique, c'est-à-dire quelle est capable de réaliser de façon automatisée la fabrication d'une lentille à partir de données numériques représentatives de la forme de la lentille à fabriquer qui lui sont transmises en entrée.

Il est à noter qu'une machine d'usinage présente les limites suivantes. Tout d'abord, le rayon d'outil utilisé impose une limite de courbure des surfaces sur la lentille dans la direction radiale. D'autre part, l'amplitude et l'accélération maximale de la vibration asymétrique imprimée à la lentille limitent respectivement l'écart par rapport à la symétrie de rotation et la courbure des surfaces dans la direction tangentielle. Enfin, des défauts de linéarité apparaissent entre les données d'entrée fournies à la machine par l'unité de calcul 3 (représentative de la forme calculée et souhaitée de la lentille) et la forme de lentilles effectivement obtenues par l'usinage.

Il est préférable de prendre en compte ces différentes limites dans le calcul des faces antérieure et postérieure de la lentille, c'est-à-dire en pratique dans le logiciel de fonctionnement de l'unité de calcul 3, afin d'une part d'éviter de commander à l'unité de fabrication 4 la fabrication d'une lentille dont les faces présentent des géométries qui seraient irréalisables et d'autre part de compenser les erreurs systématiques générées par la machine d'usinage.

Les différents éléments du dispositif peuvent être situés dans des lieux différents et reliés entre eux par un réseau.

Le fonctionnement du dispositif qui vient d'être décrit, conformément au procédé selon l'invention, se déroule de la manière suivante.

L'unité de mesure 1 de la topographie de la cornée et l'unité de mesure 2 des aberrations de l'oeil sont successivement placées devant l'oeil E à corriger pour effectuer leurs mesures respectives. Pour ces mesures, les deux unités 1 et 2 utilisent un référentiel spatial commun (non représenté sur les figures) comportant les trois axes suivants : un premier axe confondu avec la ligne principale de visée de l'oeil (droite passant par le centre de la pupille et par le point de fixation de l'instrument), un second axe qui est horizontal et orthogonal au premier axe, et un troisième axe orthogonal au premier et second axes. Bien entendu, l'utilisation de ce référentiel spatial commun suppose que les deux unités de mesure 1 et 2 comportent chacune un point de fixation et la possibilité de déterminer la position du centre de la pupille et du point de fixation par rapport aux autres données mesurées.

Après avoir effectué leurs mesures, les unités de mesure 1 et 2 délivrent chacune des données numériques représentatives du résultat de ces mesures. L'unité de mesure 1 délivre ainsi des données numériques représentatives de la topographie mesurée de la cornée de l'oeil E, tandis que l'unité de mesure 2 délivre des données numériques représentatives des aberrations oculaires de l'oeil E. Ces données sont transmises à l'unité électronique de calcul 3, comme cela est illustré par les flèches F1 et F2 à la figure 1.

A partir de ces données, l'unité électronique de calcul 3 détermine par le calcul la forme des faces antérieure et postérieure de la lentille.

Plus précisément, pour déterminer la forme de la lentille, l'unité électronique de calcul 3 procède de la manière suivante. A partir des données relatives à la topographie de la cornée de l'oeil E1 qui lui ont été fournies par l'unité de mesure 1, l'unité électronique de calcul 3 calcule la forme de la face postérieure de la lentille de manière à obtenir un comportement mécanique prédéterminé de cette lentille lorsqu'elle est installée sur l'oeil. A cet effet, la configuration calculée de la face postérieure de la lentille peut correspondre, soit exactement à celle de la surface cornéenne, soit à une transformation mathématique de cette surface. On pourra par exemple procéder comme explicité dans le brevet US 5 570 142.

La forme de la face postérieure de la lentille pourra par exemple être conçue de façon à ce qu'il existe un film de larmes équi-épais entre la lentille de la cornée. Dans un autre mode de réalisation, cette face pourra être conçue de façon à ce que l'épaisseur du film de larmes augmente lorsque la distance par rapport au centre de la lentille augmente.

A ce stade on pourra donner la possibilité au clinicien de choisir la transformation mathématique utilisée pour déduire la forme de la face arrière à partir de la topographie de la cornée.

Quel que soit le mode de calcul retenu, la forme de la face postérieure de la lentille a pour but d'éviter, ou tout au moins de maîtriser, c'est-à-dire de les identifier et de les quantifier pour les prendre en compte, les déformations de la lentille sur l'oeil ainsi que les irrégularités de l'épaisseur de larme entre la cornée et la lentille. De cette manière, la forme finale de la face postérieure de la lentille installée sur l'oeil et l'épaisseur de larme sont prédéterminées et conservées en mémoire par l'unité électronique de calcul 3.

L'unité électronique de calcul 3 détermine ensuite par le calcul la forme de la face antérieure de la lentille afin de conférer à la lentille les caractéristiques de correction optique souhaitées. Ce calcul de la forme de la face antérieure de la lentille s'effectue à partir d'une part des données relatives aux aberrations optiques de l'oeil E fournies par l'unité de mesure 2, et d'autre part des données relatives à la forme précédemment calculée de la face postérieure de la lentille. Il est en effet indispensable de combiner les données relatives à la forme donnée à la face postérieure de la lentille aux données relatives à la mesure de l'aberration de l'oeil pour calculer la forme de la face antérieure de la lentille, dans la mesure où la correction optique réalisée par la lentille dépend de la forme des deux faces, antérieure et postérieure, de la lentille, ainsi que de l'épaisseur de larme (éventuellement irrégulière) située entre la lentille et la cornée. Le cas échéant, pour ce calcul, l'unité électronique de calcul 3 exploite les données qu'elle a précédemment mémorisées sur la forme in vivo, après une éventuelle déformation de la lentille, de la face postérieure de cette lentille et sur l'épaisseur de larme qui en résulte.

Par ailleurs, dans son calcul des formes des faces antérieure et postérieure de la lentille, l'unité électronique de calcul 3 peut prendre en compte un certain nombre de données additionnelles qui lui sont transmises par saisie manuelle d'un opérateur ou sous la forme d'un fichier de données numériques, comme symbolisé par la flèche Fadd.

Ainsi, pour la détermination de la forme de la face antérieure de la lentille, il est tenu compte de la réfraction subjective du patient dont l'oeil doit être corrigé. Plus précisément, la puissance de réfraction subjective est substituée par calcul à la puissance de réfraction objective contenue dans les données résultant de la mesure des aberrations oculaires de l'oeil.

De même, pour la détermination de la forme de la face antérieure de la lentille, il est tenu compte de l'indice de réfraction du matériau constitutif de la lentille.

Egalement pour la détermination de la forme de la face antérieure de la lentille, il est tenu compte de données additionnelles de correction optique spécifique qui se combinent aux aberrations optiques mesurées. En particulier, dans le cas où l'oeil à corriger souffre de presbytie, les données additionnelles introduisent une correction complexe multifocale.

D'autre part, pour la détermination de la forme de la face postérieure de la lentille, il est tenu compte des caractéristiques mécaniques du matériau constitutif de la lentille, et en particulier des constantes d'élasticité et de viscosité de ce matériau.

Enfin, comme mentionné précédemment, pour les déterminations des configurations des faces antérieure et postérieure de la lentille, il est tenu compte des moyens prévus pour fabriquer la lentille et des limites qu'imposent ces moyens sur la forme possible des faces antérieure et postérieure de la lentille.

Après ces calculs, l'unité électronique de calcul 3 fournit un fichier de données numériques représentatif des configurations des faces antérieure et postérieure de la lentille. Comme cela est illustré par la flèche F3 de la figure 1, ce fichier est transmis à l'unité de fabrication 4. Cette dernière fabrique de façon automatisée une lentille de contact L1 dont les faces antérieure et postérieure sont conformes aux données contenues dans le fichier transmis.

La figure 2 illustre une étape supplémentaire du procédé visant à affiner la forme de lentilles initialement déterminées comme expliqué ci-dessus.

La première lentille L1 qui a été déterminée comme indiqué précédemment est utilisée comme lentille d'essai. Cette lentille L1 est ainsi placée sur l'oeil E à corriger et une nouvelle mesure des aberrations optiques du système optique constitué par l'oeil E et la lentille L1 est effectuée au moyen de l'unité de mesure 2. Le résultat de ces mesures d'aberrations optiques est fourni par l'unité de mesure 2 sous la forme d'un fichier de données numériques et est transmis à l'unité électronique de calcul 3 comme illustré par la flèche F2' à la figure 2.

L'unité électronique de calcul 3 recalcule alors la configuration de la face antérieure de la lentille à partir d'une part des données qui lui sont fournies par l'unité de mesure 2 des aberrations optiques et d'autre part des données relatives à la forme initialement déterminée de la lentille L1 qu'elle aura mémorisées. L'unité électronique de calcul 3 fournit alors un fichier de données numériques représentatives de la forme corrigée de la face antérieure de la lentille et de la forme de la face postérieure de la lentille qui, elle, reste inchangée. Ce fichier est transmis à l'unité de fabrication 4 comme cela est symbolisé par la flèche F3' à la figure 2.

L'unité de fabrication 4 fabrique alors une lentille L2 dont la face antérieure est corrigée par rapport à celle de la lentille d'essai L1.

Les lentilles obtenues ont préférentiellement des moyens de stabilisation du type ballast, troncature inférieure, bossages palpébraux du type de ceux décrits dans le brevet de la demanderesse FR 2 760 853, allègements supérieur et inférieur ou une combinaison de ces moyens. Les lentilles obtenues auront avantageusement des marquages en dehors de la zone optique. On pourra à titre d'exemple se référer au brevet FR 2 777 093 de la demanderesse.

Ces marquages permettent au clinicien de vérifier le positionnement convenable de la lentille. Ils indiquent par ailleurs un repère de référence pour les instruments d'inspection des lentilles.

La figure 3 illustre un mode de réalisation simplifié d'un dispositif de fabrication de lentilles de contact utilisant un dispositif et un procédé de détermination de la forme de lentilles selon l'invention. Ce mode de réalisation simplifié vise les cas où les aberrations oculaires de l'oeil à corriger proviennent essentiellement de défauts de forme de la cornée. Dans ses éléments essentiels, le dispositif de la figure 3 correspond au dispositif de la figure 1 et les mêmes éléments sont désignés par les mêmes références numériques. Selon ce mode de réalisation, les aberrations optiques de l'oeil E ne sont plus mesurées en tant que telles par une unité de mesure (qui est donc ici inexistante), mais sont simplement déduites, par un calcul effectué par les moyens logiciels de l'unité électronique de calcul 3, de la topographie cornéenne mesurée par l'unité de mesure 1. Les autres parties du dispositif et étapes du procédé sont identiques à celles précédemment décrites en référence aux figures 1 et 2.

## Revendications

1. Procédé pour déterminer la forme d'une lentille de contact ophtalmique de correction des aberrations optiques d'un oeil, comportant :
- une étape de mesure des aberrations optiques de l'oeil à corriger, et
- une étape de détermination de la forme de la face antérieure de ladite lentille à partir de la mesure des aberrations optiques de l'oeil à corriger pour corriger ces aberrations,
caractérisé en ce qu'il comporte :
- une étape de mesure de la topographie de la cornée de l'oeil à corriger, et
- une étape de détermination de la forme de la face postérieure de la lentille à partir de la topographie mesurée de la cornée pour limiter la génération d'aberrations parasites lorsque la lentille est posée sur l'oeil,
et en ce que, pour la détermination de la forme de la face antérieure de la lentille, des données relatives à la forme déterminée pour la face postérieure de la lentille sont combinées à la mesure des aberrations optiques de l'oeil.

2. Procédé selon la revendication 1, caractérisé en ce que la mesure de la topographie de la cornée de l'oeil à corriger et la mesure des aberrations dudit oeil à corriger sont réalisées dans un référentiel spatial commun.

3. Procédé selon la revendication 2, caractérisé en ce que le référentiel spatial commun possède un premier axe confondu avec la ligne principale de visée de l'oeil à corriger.

4. Procédé selon la revendication 3, caractérisé en ce que le référentiel spatial commun comporte un second axe horizontal et orthogonal au premier axe et un troisième axe orthogonal aux premier et second axes.

5. Procédé selon l'une des revendications précédentes, caractérisé en ce que, pour affiner la forme de la lentille initialement déterminée, il comporte :
- une étape de fabrication d'une lentille d'essai ayant des faces antérieure et postérieure conformes aux déterminations obtenues à l'issue des étapes précitées,
- une étape de mesure in vivo des aberrations optiques de l'ensemble optique constitué par l'oeil à corriger équipé de la lentille d'essai fabriquée, et
- une étape de correction de la forme de la face antérieure de la lentille à partir de données relatives à la forme initialement déterminée de la lentille d'essai et de la nouvelle mesure des aberrations optiques de l'oeil avec la lentille d'essai.

6. Procédé selon l'une des revendications précédentes, caractérisé en ce que, pour la détermination de la forme de la face antérieure de la lentille, il est tenu compte de la réfraction subjective du patient dont l'oeil doit être corrigé.

7. Procédé selon la revendication 6, caractérisé en ce que la puissance de réfraction subjective est substituée par calcul à la puissance de réfraction objective contenue dans les données résultant de la mesure des aberrations oculaires de l'oeil.

8. Procédé selon l'une des revendications précédentes, caractérisé en ce que, pour la détermination de la forme de la face antérieure de la lentille, il est tenu compte de l'indice de réfraction du matériau constitutif de la lentille.

9. Procédé selon l'une des revendications précédentes, caractérisé en ce que, pour la détermination de la forme de la face antérieure de la lentille, il est tenu compte de données additionnelles de correction optique spécifique qui se combinent aux aberrations optiques mesurées.

10. Procédé selon la revendication 9, caractérisé en ce que, dans le cas où l'oeil à corriger souffre de presbytie, les données additionnelles introduisent une correction complexe multifocale.

11. Procédé selon l'une des revendications précédentes, caractérisé en ce que, pour la détermination de la forme de la face postérieure de la lentille, il est tenu compte des caractéristiques mécaniques du matériau constitutif de la lentille.

12. Procédé selon la revendication 11, caractérisé en ce que les caractéristiques mécaniques du matériau constitutif de la lentille dont il est tenu compte sont les constantes d'élasticité et de viscosité de ce matériau.

13. Procédé selon l'une des revendications précédentes, caractérisé en ce que, pour les déterminations des configurations des faces antérieure et postérieure de la lentille, il est tenu compte des moyens prévus pour fabriquer la lentille et des limites qu'imposent ces moyens sur la forme possible des faces antérieure et postérieure de la lentille.

14. Procédé selon l'une des revendications précédentes, caractérisé en ce que l'étape de mesure des aberrations de l'oeil à corriger se confond avec l'étape de mesure de la topographie de la cornée dudit oeil à corriger, ces aberrations étant déduites par le calcul de la topographie mesurée de ladite cornée.

15. Dispositif pour la mise en oeuvre d'un procédé selon l'une des revendications 1 à 13, caractérisé en ce qu'il comporte :
- une unité de mesure (1) de la topographie de la cornée de l'oeil (E) à corriger, délivrant des données numériques représentatives de cette topographie,
- une unité de mesure (2) des aberrations de l'oeil (E) à corriger, délivrant des données numériques représentatives de ces aberrations,
- une unité électronique de calcul (3) apte à déterminer, à partir des données qui lui sont délivrées d'une part par l'unité de mesure (1) de la topographie de la cornée de l'oeil à corriger et d'autre part par l'unité de mesure (2) des aberrations dudit oeil à corriger, les formes des faces antérieure et postérieure de la lentille, et à délivrer des données numériques représentatives de ces formes.

16. Dispositif selon la revendication 15, caractérisé en ce que les unités de mesure de la topographie de la cornée et des aberrations utilisent un référentiel spatial commun.

17. Dispositif selon la revendication 16, caractérisé en ce que le référentiel spatial commun possède un premier axe confondu avec la ligne principale de visée de l'oeil à corriger.

18. Dispositif selon la revendication 17, caractérisé en ce que le référentiel spatial commun comporte un second axe horizontal et orthogonal au premier axe et un troisième axe orthogonal aux premier et second axes.

19. Dispositif pour la mise en oeuvre d'un procédé selon la revendication 14, caractérisé en ce qu'il comporte :
- une unité de mesure (1) de la topographie de la cornée de l'oeil (E) à corriger, délivrant des données numériques représentatives de cette topographie,
- une unité électronique de calcul (3) apte à estimer les aberrations produites par la cornée à partir des données délivrées par l'unité de mesure (1) de la topographie de la cornée, à déterminer les formes des faces antérieure et postérieure de la lentille à partir des données qui lui sont délivrées par l'unité de mesure (1) de la topographie de la cornée et des aberrations estimées et à délivrer des données numériques représentatives de ces formes.

20. Dispositif selon l'une des revendications 15 à 19, caractérisé en ce qu'il comporte une unité de fabrication (4) d'une lentille (L1, L2) à partir des données qui lui sont délivrées par l'unité électronique de calcul (3).
